# EUROPÄISCHE PATENTSCHRIFT

(11) **EP 4 076 606 B1**
(45) Veröffentlichungstag und Bekanntmachung des Hinweises auf die Patenterteilung: **01.05.2024**
(21) Anmeldenummer: 20838356.2
(22) Anmeldetag: 09.12.2020
(51) Int. Cl.: A61M 16/00, A61M 16/10, A61M 16/16

(54) **TRAGBARES BEATMUNGSGERÄT MIT STROMVERSORGUNG DURCH STROMNETZ UND STROMSPEICHER**
PORTABLE RESPIRATORY DEVICE HAVING POWER SUPPLY VIA POWER GRID AND ELECTRICITY STORE
DISPOSITIF RESPIRATOIRE PORTABLE POURVU D'UNE ALIMENTATION BASÉE SUR LE RÉSEAU ÉLECTRIQUE ET SUR UN ACCUMULATEUR D'ÉLECTRICITÉ

(30) Priorität: 17.12.2019 DE 102019134830
(43) Veröffentlichungstag der Anmeldung: 26.10.2022
(73) Patentinhaber: Hamilton Medical AG, 7402 Bonaduz (CH)
(72) Erfinder: SAIHI, Kaouther, 7000 Chur (CH); BARANDUN, Daniel, 7000 Chur (CH); SINOGOWITZ, Niko, 7023 Haldenstein (CH); ZÄCH, Daniel, 7000 Chur (CH); WITTON, Gabriele, 7000 Chur (CH)
(74) Vertreter: Ruttensperger Lachnit Trossin Gomoll
(86) Internationale Anmeldenummer: PCT/EP2020/085254
(87) Internationale Veröffentlichungsnummer: WO 2021/122212

(56) Entgegenhaltungen:
- WO-A1-2017/127823
- WO-A1-2019/070135
- WO-A1-2019/075510
- WO-A2-2007/117716

## Beschreibung

Die Erfindung ist im unabhängigen Anspruch 1 definiert. Bevorzugte Ausführungsformen sind Gegenstand der abhängigen Ansprüche.

Derartige tragbare Beatmungsgeräte sind vor allen Dingen aus der sogenannten "High-Flow-Beatmung" bekannt, gemäß welcher einem Patienten unabhängig von dessen Atemrhythmus ein im Wesentlichen konstant hoher Atemgasstrom zugeführt wird. Der Atemgasstrom beträgt bis zu 100 Liter pro Minute. Der Atemgasstrom wird in der Regel über Nasenkanülen in den Bereich der Nasenöffnungen abgegeben oder über eine Nasen-Mund-Maske oder über eine Trachealkanüle verabreicht, welche Nase und Mund des Patienten bedeckt. Beim Einatmen atmet der Patient somit unweigerlich wenigstens einen Teil des zugeführten Atemgasstroms ein. Das Ausatmen erfolgt gegen den üblicherweise auch während des Ausatmens zugeführten Atemgasstrom.

Derartige Beatmungsgeräte weisen üblicherweise einen Atemgasspeicher auf, beispielsweise in Form einer Atemgasflasche, in welcher Atemgas bezüglich der Atmosphäre unter Überdruck stehend aufgenommen ist. Die Atemgas-Fördervorrichtung umfasst dann die Atemgasflasche und ein Druckminderungsventil, welches das in der Atemgasflasche unter hohem Druck gespeicherte Atemgas auf einen für den Patienten verträglichen Atemgasdruck mindert.

Andere tragbare Beatmungsgeräte weisen ein Gebläse als Atemgas-Fördervorrichtung auf, wobei das Gebläse während seines Betriebs über eine Netzspannungsquelle mit Strom versorgt wird.

Einige dieser tragbaren Beatmungsgeräte sind zum Beispiel in den Dokumenten WO2007149446, WO 2007/117716, WO 2019/075510, WO 2019/070135 und WO 2017/127823 offengelegt.

Nachteilig an den bekannten Lösungen ist im ersten Fall die bei guter Mobilität durch den Flascheninhalt begrenzte Betriebsdauer und im zweiten Fall die bei langer möglicher Betriebsdauer durch die Stromversorgung per Netzspannungsquelle eingeschränkte Mobilität.

Es ist daher Aufgabe der vorliegenden Erfindung ein tragbares Beatmungsgerät der eingangs genannten Art bereitzustellen, welches sowohl eine lange ununterbrochene Betriebsdauer als auch eine möglichst uneingeschränkte Mobilität bietet.

Diese Aufgabe löst die vorliegende Erfindung an einem tragbaren Beatmungsgerät, wie es eingangs dieser Anmeldung beschrieben ist, dadurch, dass das Beatmungsgerät eine zweite, speicherbasierte Stromversorgung aufweist, welche einen Stromspeicher zur Speicherung elektrischer Energie aufweist, und welche wenigstens zur Versorgung der Atemgas-Fördervorrichtung mit Strom ausgebildet ist.

Durch die zweite, speicherbasierte Stromversorgung ist das tragbare Beatmungsgerät nicht ausschließlich auf einen Anschluss an eine Netzspannungsquelle angewiesen, was die Mobilität gegenüber einem tragbaren Beatmungsgerät mit ausschließlichem Netzanschluss als Energieversorgung erheblich erhöht. Somit ist es möglich, einen nahezu unbegrenzten Atemgasvorrat durch ein Gebläse als Atemgas-Fördervorrichtung zu erschließen und so Atemgas dauerhaft dem Patienten zuzuführen. Aufgrund der vorgesehenen zweiten Stromversorgung, die einen eigenen Speicher für elektrische Energie, nachfolgend als "Stromspeicher" bezeichnet, aufweist, kann der Bewegungsradius des Patienten vorübergehend gegenüber einer ausschließlichen Netzversorgung erheblich erhöht werden. Betriebsphasen, bei welchen die Atemgas-Fördervorrichtung durch eine Netzspannungsquelle, also etwa ein öffentliches Stromnetz, versorgt wird, und Betriebsphasen, bei welchen die Atemgas-Fördervorrichtung durch den Stromspeicher der zweiten Stromversorgung versorgt wird, können sich dabei abwechseln. Hierdurch wird eine größtmögliche Betriebsdauer bei gleichzeitiger möglicher Mobilität erreicht. So kann sich der beatmete Patient beispielsweise innerhalb eines Krankenhauses vom Stromnetz unabhängig zwischen unterschiedlichen Behandlungs- oder/und Diagnosestationen bewegen.

Bevorzugt umfasst daher die Atemgas-Fördervorrichtung ein Gebläse, welches Luft aus der Umgebung des Beatmungsgeräts ansaugt und zu dem Atemgas-Gehäuseausgang fördert. Im Betrieb ist an den Atemgas-Gehäuseausgang üblicherweise eine Atemgas-Förderleitung, bevorzugt ein flexibler Atemgas-Schlauch, angeschlossen, um Atemgas vom Gehäuse zum Patienten zu leiten. Bevorzugt ist das Beatmungsgerät ein eingangs genanntes High-Flow-Beatmungsgerät.

Das Beatmungsgerät kann zusätzlich zum Gebläse eine Anschlussformation, beispielsweise einen Anschlussstutzen oder eine Schnellkupplung, zum Anschluss eines von der Umgebungsluft verschiedenen, weiteren Atemgasvorrats aufweisen. So kann beispielsweise der vom Gebläse angesaugten Umgebungsluft reiner Sauerstoff oder ein anderes gewünschtes Gas, eine Gasmischung oder ein Aerosol-haltiges Gas beigemischt werden. Die vom Gebläse angesaugte Umgebungsluft wird daher bevorzugt über eine Mischkammer oder eine Misch-Leitungsstrecke geführt, in die auch eine von der Anschlussformation kommende weitere Leitung mündet, bevor sie den Atemgas-Gehäuseausgang erreicht.

Die Speicherkapazität des Stromspeichers der zweiten Stromversorgung ist vorzugsweise so dimensioniert, dass ein Beatmungsbetrieb nur durch die zweite Stromversorgung wenigstens über eine Stunde, vorzugsweise über mehrere Stunden aufrechterhalten werden kann.

Für die Versorgungssicherheit des Patienten mit Atemgas reicht es grundsätzlich aus, wenn die zweite Stromversorgung ausschließlich die Atemgas-Fördervorrichtung mit elektrischer Energie versorgt. Bevorzugt dient die zweite Stromversorgung jedoch über die bloße Versorgung des Patienten mit Atemgas hinaus auch der möglichst umfangreichen Steuer- und Einstellbarkeit der Atemgas Versorgung des Patienten. Deshalb ist bevorzugt vorgesehen, dass die zweite Stromversorgung auch zur Versorgung der Steuervorrichtung und der Eingabe/Ausgabevorrichtung mit Strom ausgebildet ist. Besonders bevorzugt dient die zweite Stromversorgung der Versorgung derselben Funktionsvorrichtungen innerhalb des Beatmungsgeräts wie die erste Stromversorgung, sodass die zweite Stromversorgung gemäß dieser besonders bevorzugten Ausgestaltung die erste Stromversorgung vollumfänglich ersetzen kann.

Auch in diesem Fall gilt, dass bevorzugt der Stromspeicher so dimensioniert ist, dass der Betrieb des Beatmungsgeräts nur durch die zweite Stromversorgung wenigstens über eine Stunde, vorzugsweise über mehrere Stunden aufrechterhalten werden kann.

Zur Konditionierung des Atemgases hinsichtlich seines Feuchtigkeitsgehalts kann das Beatmungsgerät gemäß einer bevorzugten Weiterbildung der vorliegenden Erfindung eine Befeuchtungsvorrichtung aufweisen, welche dazu ausgebildet ist, das inspiratorische Atemgas zu befeuchten. Dabei ist ebenso bevorzugt die Befeuchtungsvorrichtung sowohl durch die erste als auch durch die zweite Stromversorgung mit Strom versorgbar. Dies bedeutet, die Befeuchtungsvorrichtung kann entweder durch die erste oder durch die zweite Stromversorgung mit Strom versorgbar sein. Beispielsweise kann die Befeuchtungsvorrichtung eine Heizplatte oder eine andere Verdampfungsvorrichtung aufweisen, welche elektrisch betrieben Wasser oder eine wässrige Lösung verdampft und so dem inspiratorischen Atemgas zuführt.

Eine Atemgasleitung, die vom Beatmungsgerät zum Patienten führt, ist vorzugsweise zur Vermeidung von Kondensation in der Atemgasleitung beheizbar. Auch diese Heizleistung einer Heizvorrichtung der Atemgasleitung kann wahlweise von der ersten oder von der zweiten Stromversorgung zugeführt werden. Da elektrisches Heizen sehr energieintensiv ist, kann bei Stromversorgung des Beatmungsgeräts durch die zweite Stromversorgung bevorzugt die Heizvorrichtung der Atemgasleitung isoliert abgeschaltet werden, um elektrische Energie zu sparen und so die Betriebsdauer der zweiten Stromversorgung zu verlängern.

Der Stromspeicher der zweiten Stromversorgung ist bevorzugt wiederaufladbar. Hierzu kann die zweite Stromversorgung mit einem Ladegerät elektrisch leitend verbindbar sein, welches selbst wiederum mit einem Lade-Stromspeicher oder eine Netzspannungsquelle verbindbar ist. Mit dem Ziel, die Anzahl an benötigten Vorrichtungen für einen Betrieb oder für die Herstellung eines betriebsbereiten Zustands des Beatmungsgeräts möglichst gering zu halten, ist bevorzugt der Stromspeicher der zweiten Stromversorgung durch die erste Stromversorgung aufladbar. Dies ist ohne weiteres möglich, da die erste Stromversorgung mit der Netzspannungsquelle verbindbar ist, sodass aus der Netzspannungsquelle ausreichend elektrische Energie für ein Aufladen des Stromspeichers der zweiten Stromversorgung zur Verfügung steht. Um ein unkontrolliertes Laden des Stromspeichers der zweiten Stromversorgung und damit möglicherweise ein Überladen desselben zu vermeiden, ist bevorzugt die Steuervorrichtung dazu ausgebildet, einen Ladevorgang des Stromspeichers durch die erste Stromversorgung zu steuern. Hierzu kann die Steuervorrichtung kontinuierlich oder mit Unterbrechungen den Ladezustand des Stromspeichers abfragen und abhängig vom Abfrageergebnis den Ladevorgang beenden.

Um gewährleisten zu können, dass bei Ausfall der Netzspannungsquelle, etwa während eines Blackouts der öffentlichen Stromversorgung oder nach lokaler Abschaltung der Netzversorgung durch Aktivierung einer elektrischen Sicherung, wenigstens die Atemgas-Fördervorrichtung ohne Gefährdung des Patienten weiter an diesem Atemgas fördert, vorzugsweise das gesamte Beatmungsgerät in Betrieb bleibt, ist bevorzugt vorgesehen, dass die zweite Stromversorgung als unterbrechungsfreie Stromversorgung für den Fall eines Endes einer Stromversorgung des Beatmungsgeräts durch die erste Stromversorgung ausgebildet ist. Dabei soll eine Unterbrechung der Stromversorgung beim Umschalten von der ersten auf die zweite Stromversorgung von bis zu 50 ms fachüblich als "unterbrechungsfrei" gelten. Wegen der medizinischen Anwendung bevorzugt, setzt die zweite Stromversorgung im Falle eines Versorgungsendes der ersten Stromversorgung im buchstäblichen Sinne die Stromversorgung wenigstens der Atem-Gas-Fördervorrichtung, vorzugsweise auch anderer Funktionsvorrichtungen, unterbrechungsfrei fort, sodass die Stromversorgung durch die zweite Stromversorgung nahtlos auf eine Stromversorgung durch die erste Stromversorgung folgt.

Grundsätzlich kann die zweite Stromversorgung im Gehäuse des Beatmungsgeräts aufgenommen sein. Da üblicherweise Stromspeicher, wie sie die zweite Stromversorgung benötigt, aufgrund ihrer überdurchschnittlich großen Dichte ein hohes Gewicht aufweisen, ist zur Begrenzung des Gewichts des tragbaren Beatmungsgeräts und damit zur Verbesserung der Beweglichkeit des Beatmungsgeräts bevorzugt daran gedacht, dass die zweite Stromversorgung ein vom Gehäuse des Beatmungsgeräts gesondertes Stromversorgungsgehäuse aufweist. Somit kann die zweite Stromversorgung dann, wenn sie nicht benötigt wird, vom Gehäuse des Beatmungsgeräts getrennt werden. Dies ist beispielsweise in Betriebsphasen vorstellbar, in denen das Beatmungsgerät ausschließlich durch die erste Stromversorgung mit elektrischer Energie versorgt wird. Deshalb ist gemäß einer bevorzugten Ausführungsform das Stromversorgungsgehäuse der zweiten Stromversorgung körperlich lösbar mit dem Gehäuse des Beatmungsgeräts zur gemeinsamen Bewegung koppelbar. Wenn also die zweite Stromversorgung körperlich mit dem Gehäuse des Beatmungsgeräts gekoppelt ist, ist es auch gemeinsam mit diesem transportierbar, sodass nur ein Gegenstand bewegt werden muss, welcher das Gehäuse und das Stromversorgungsgehäuse umfasst. Derjenige, der dann das Beatmungsgerät bewegt, hat so immer noch eine Hand frei.

Auf das überdurchschnittlich hohe Gewicht pro Volumeneinheit des Stromspeichers der zweiten Stromversorgung und damit der zweiten Stromversorgung insgesamt wurde oben bereits hingewiesen. Um sicherzugehen, dass das vorliegend diskutierte Beatmungsgerät eine möglichst hohe Standsicherheit aufweist, ist die zweite Stromversorgung bei betriebsbereiter Aufstellung des Beatmungsgeräts bevorzugt in den untersten 20 % der Gesamthöhenerstreckung des Beatmungsgeräts mit zweiter Stromversorgung angeordnet. Angesichts der oben angesprochenen bevorzugten körperlichen Koppelbarkeit, die die Trennbarkeit einschließt, von Gehäuse und Stromversorgungsgehäuse ist bevorzugt die zweite Stromversorgung die unterste Funktionsvorrichtung an dem die zweite Stromversorgung umfassenden Beatmungsgerät, um nicht nur einen tief liegenden, sondern einen so tief wie möglich liegenden Schwerpunkt des Beatmungsgeräts zu erzeugen. Der Gesichtspunkt des möglichst tief liegenden Schwerpunkts des Beatmungsgeräts gilt sowohl für den Fall, dass die zweite Stromversorgung mit dem Gehäuse des Beatmungsgeräts koppelbar und von diesem trennbar ist, als auch für den Fall, dass die zweite Stromversorgung im Gehäuse des Beatmungsgeräts aufgenommen ist.

Da die oben genannte Befeuchtungsvorrichtung einen Flüssigkeitsvorrat und damit ebenfalls ein überdurchschnittlich großes Gewicht pro Volumeneinheit aufweisen kann, sind vorzugsweise die Befeuchtungsvorrichtung, wenigstens jedoch ihr Flüssigkeitsvorrat, und die zweite Stromversorgung in unmittelbarer Nachbarschaft angeordnet. Bevorzugt sind die Befeuchtungsvorrichtung, wenigstens jedoch ihr Flüssigkeitsvorrat, und die zweite Stromversorgung bei Betrachtung des betriebsbereit hergerichteten Beatmungsgeräts übereinander angeordnet, wobei bevorzugt die zweite Stromversorgung unter der Befeuchtungsvorrichtung angeordnet ist.

Um am abgestellten Beatmungsgerät unerwartete Stolperfallen zu vermeiden, ist bevorzugt das Stromversorgungsgehäuse - bei Betrachtung einer betriebsbereiten Aufstellung des Beatmungsgeräts - vollständig innerhalb eines in Schwerkraftwirkungsrichtung projizierten Grundrisses des Gehäuses des Beatmungsgeräts aufgenommen. Somit ragt das Stromversorgungsgehäuse seitlich nicht über das Gehäuse des Beatmungsgeräts hinaus vor. Aus Gründen möglichst hoher Standsicherheit bleibt bevorzugt das Stromversorgungsgehäuse dann, wenn es die unterste Funktionseinheit des Beatmungsgeräts bildet, auch nicht hinter dem in Schwerkraftwirkungsrichtung projizierten Grundriss des Gehäuses des Beatmungsgeräts zurück. Bevorzugt bilden das Gehäuse und das Stromversorgungsgehäuse mit Ausnahme einer unvermeidlichen Trennfuge zwischen ihnen eine bündige Außenwand der Gesamtvorrichtung aus Gehäuse und Stromversorgungsgehäuse.

Da zur Erzielung eines möglichst tiefen Schwerpunkts die zweite Stromversorgung bevorzugt die bei betriebsbereiter Aufstellung des Beatmungsgeräts unterste Funktionsvorrichtung des Beatmungsgeräts ist, die aus den oben genannten Gründen ebenso bevorzugt unter einem Flüssigkeitsvorrat einer Befeuchtungsvorrichtung angeordnet ist, kann es vorkommen, dass Flüssigkeit - sei es als Leckage aus dem Flüssigkeitsvorrat, sei es als Kondenswasser - am Gehäuse schwerkraftgetrieben herunterlaufend, das Stromversorgungsgehäuse erreicht.

Zur Vermeidung von Schäden durch Feuchtigkeit an der zweiten Stromversorgung ist das Stromversorgungsgehäuse bevorzugt feuchtigkeitsdicht ausgebildet.

Zur Vermeidung von Feuchtigkeitsschäden an der Aufstellumgebung des Beatmungsgeräts, insbesondere am Aufstell-Untergrund, auf dem das Beatmungsgerät im Betrieb steht, sind am Stromversorgungsgehäuse bevorzugt Ableitflächen ausgebildet, die darauf auftreffende Flüssigkeit gezielt zu wenigstens einem definierten Ableitort ableiten, wo die abgeleitete Flüssigkeit erwartet und aufgefangen werden kann.

Die Ableitflächen umfassen bevorzugt wenigstens zwei unterschiedlich geneigte Flächenabschnitte, welche - in betriebsbereiter Orientierung des Beatmungsgeräts mit angekoppelter zweiter Stromversorgung - in Schwerkraftwirkungsrichtung aufeinander folgen. Dabei führt bevorzugt ein erster Flächenabschnitt aufgrund seiner ersten Neigung Flüssigkeit von der Außenwand des Beatmungsgeräts nach innen in einen Spaltraum zwischen Gehäuse und Stromversorgungsgehäuse zu einem in Richtung der ersten Neigung auf den ersten Flächenabschnitt folgenden zweiten Flächenabschnitt, welcher aufgrund seiner zweiten Neigung die an ihn vom ersten Flächenabschnitt übergebene Flüssigkeit zu dem Ableitort ableitet. Der Ableitort ist bevorzugt an einer Außenfläche des Beatmungsgeräts gelegen, bevorzugt an der Trennfuge zwischen Gehäuse und Stromversorgungsgehäuse. Um Störungen des Betriebs und der Betätigung des Beatmungsgeräts durch die abgeleitete Flüssigkeit zu vermeiden, ist der Ableitort bevorzugt nicht an der Vorderseite des Beatmungsgeräts, besonders bevorzugt an dessen Rückseite. Dort kann durch ein am Abgabeort bereitgestelltes saugfähiges Tuch oder einen ebensolchen Schwamm die abgeleitete Flüssigkeit aufgefangen werden.

Da die Flüssigkeitsableitung an einer Oberfläche des Stromversorgungsgehäuses erfolgt, sind der erste und der zweite Flächenabschnitt bevorzugt als Abschnitte einer Oberfläche des Stromversorgungsgehäuses ausgebildet, welche im an das Gehäuse angekoppelten Zustand einen Trennspalt zwischen Gehäuse und Stromversorgungsgehäuse aufseiten des Stromversorgungsgehäuses begrenzt.

Für eine möglichst großflächige Ableitung von Flüssigkeit läuft bevorzugt der erste Flächenabschnitt wenigstens abschnittsweise um das Gehäuse, wegen der schwerkraftgetriebenen Ableitung bevorzugt um dessen Höhenachse um. Bevorzugt erstreckt sich der erste Flächenabschnitt auf der Vorderseite und auf den beiden daran zu unterschiedlichen Seiten anschließenden Seitenflächen des Stromversorgungsgehäuses. Der erste Flächenabschnitt kann in Richtung zu einer Körpermitte des Stromversorgungsgehäuses hin geneigt sein, um Flüssigkeit von der Außenfläche des Beatmungsgeräts nach innen in das Trennspaltvolumen zwischen Gehäuse und Stromversorgungsgehäuse leiten zu können. Der erste Flächenabschnitt kann um eine Krümmungsachse gekrümmt sein, um eine möglichst große Länge längs der Außenwandung des Stromversorgungsgehäuses erzielen zu können. Die Krümmungsachse ist daher bevorzugt von der Neigeachse oder den Neigeachsen des ersten Flächenabschnitts verschieden.

Der zweite Flächenabschnitt schließt bevorzugt unmittelbar an den ersten Flächenabschnitt an. Er kann im einfachsten Fall eine ebene geneigte Fläche sein, an dessen geodätisch tiefstem Punkt der Ablenkort gelegen ist.

Um eine möglichst geringe Standfläche zu erfordern, ist das Gehäuse bevorzugt ein Turmgehäuse, dessen größte Abmessung bei betriebsbereiter Aufstellung die Höhenabmessung ist. Vorzugsweise ist das Gehäuse wenigstens eineinhalb mal so hoch wie es breit ist und ebenso wenigstens eineinhalb mal so hoch wie es tief ist. "Breite" und "Tiefe" sind dabei sowohl zueinander als auch zur Höhenrichtung des Gehäuses orthogonale Abmessungsrichtungen.

Zur leichteren Bedienung kann das Gehäuse die Eingabe/Ausgabevorrichtung an seinem oberen Endbereich aufweisen. Eine besonders gute Zugänglichkeit von Bedieneinheiten der Eingabe/Ausgabevorrichtung kann dadurch erhalten werden, dass die Eingabe/Ausgabevorrichtung eine sowohl zur Höhenrichtung als auch zur Tiefenrichtung um eine in Breitenrichtung verlaufende Anstellachse geneigte oder/und gekrümmte Eingabe/Ausgabefläche aufweist, an welcher Bedieneinheiten, wie etwa Schaltflächen, Schalter, Anzeigeflächen und dergleichen, angeordnet oder ausgebildet sind.

Die Steuervorrichtung ist zur Erzielung einer möglichst langen Betriebsdauer nur mit Energie aus der zweiten, speicherbasierten Stromversorgung dazu ausgebildet, den Betrieb des Beatmungsgeräts, insbesondere einer Heizvorrichtung, bevorzugt aller Heizvorrichtungen im und am Beatmungsgerät, stärker bevorzugt der oben genannten Heizplatte, nach Maßgabe eines Erfassungssignals eines mit der Steuervorrichtung signalübertragungsmäßig verbundenen Temperatursensors derart zu steuern bzw. zu regeln, dass das vom Temperatursensor an die Steuervorrichtung übertragene Temperatursignal eine Temperatur repräsentiert, welche eine vorbestimmte Grenztemperatur nicht übersteigt. Die vorbestimmte Grenztemperatur kann im Bereich von 30 °C bis 40 °C liegen und liegt bevorzugt im Bereich von 33 °C bis 38 °C, wenn sie eine Temperatur eines inspiratorischen Atemgases im Bereich des proximalen Endbereichs eines an das Beatmungsgerät angeschlossenen Atemgasschlauchs repräsentiert. Bevorzugt beträgt die Grenztemperatur 35 °C.

Das Beatmungsgerät umfasst bevorzugt wenigstens einen Temperatursensor, welcher eine Temperatur eines inspiratorischen Atemgases im Bereich des proximalen Endbereichs eines an das Beatmungsgerät angeschlossenen Atemgasschlauchs erfasst und an die Steuervorrichtung überträgt. Bevorzugt ist wenigstens ein Temperatursensor am Atemgasschlauch angeordnet, und zwar bevorzugt in einem Bereich von nicht mehr als 50 cm, vorzugsweise nicht mehr als 30 cm, vor einer Ausgabe von inspiratorischem Atemgas an den Patienten.

Zur Aufrechterhaltung eines Beatmungsbetriebs über eine möglichst lange Zeitdauer kann die Steuervorrichtung weiter dazu ausgebildet sein, abhängig vom Ladezustand des Stromspeichers nur noch vorbestimmte ausgewählte Komponenten des Beatmungsgeräts mit Strom zu versorgen.

Besonders intensive Stromverbraucher sind elektrisch betriebene Heizvorrichtungen. Daher kann die Steuervorrichtung dazu ausgebildet sein, abhängig vom Ladezustand des Stromspeichers eine oder mehrere Heizvorrichtungen stillsetzen. Das Stillsetzen kann beispielsweise durch Unterbrechung der Stromversorgung oder durch Abschaltung erfolgen. Dieses Stillsetzen kann abhängig von unterschiedlichen Grenz-Ladezuständen für unterschiedliche Komponenten gestaffelt erfolgen, wobei unterschiedlichen Komponenten betragsmäßig unterschiedliche Grenz-Ladezustände zugeordnet sind, deren Unterschreitung zur Stillsetzung der jeweiligen Komponente führt. So kann dann, wenn der Ladezustand des Stromspeichers einen ersten Grenz-Ladezustand unterschreitet, die Steuervorrichtung die Verdampfungsvorrichtung, insbesondere deren Heizplatte, stillsetzen. Die Druckveränderungsvorrichtung und andere Komponenten können weiter betrieben werden. Dann, wenn der Ladezustand einen zweiten Grenz-Ladezustand unterschreitet, welcher betragsmäßig niedriger ist als der erste Grenz-Ladezustand, kann vorgesehen sein, eine Heizvorrichtung der Atemgasleitung stillzusetzen. Wiederum können die Druckveränderungsvorrichtung und weitere Komponenten weiter betrieben werden.

Schließlich kann dann, wenn ein dritter Grenz-Ladezustand unterschritten wird, welcher betragsmäßig niedriger ist als der zweite Grenz-Ladezustand, vorgesehen sein, dass die Steuervorrichtung den Beatmungsbetrieb stillsetzt. Der dritte Grenz-Ladezustand kann der betragsmäßig niedrigste Ladezustand sein, welcher eine gezielte Stillsetzung einer oder mehrerer Komponenten durch die Steuervorrichtung bewirkt. Es kann jedoch noch einen betragsmäßig geringeren Grenz-Ladezustand geben, bis zu dessen Erreichen Datenspeicher, insbesondere volatile Datenspeicher mit Strom versorgt werden, um Daten über den Betrieb ausgeben oder in nicht-volatilen Speichermedien des Beatmungsgeräts sichern zu können.

Die vorliegende Erfindung wird nachfolgend anhand der beiliegenden Zeichnungen näher erläutert werden. Es stellt dar:
- Figur 1: eine perspektivische Ansicht von schräg vorne und oben eines erfindungsgemäßen tragbaren Beatmungsgeräts,
- Figur 2: eine Rückansicht des Beatmungsgeräts von Figur 1,
- Figur 3: eine perspektivische Ansicht von schräg vorne und oben einer zweiten Stromversorgung, wie sie in Figur 1 strichliniert angedeutet ist, und
- Figur 4: das tragbare Beatmungsgerät von Figur 1 mit daran eingesetztem Behälter mit Flüssigkeitsvorrat und mit angeschlossenem Beatmungsschlauch.

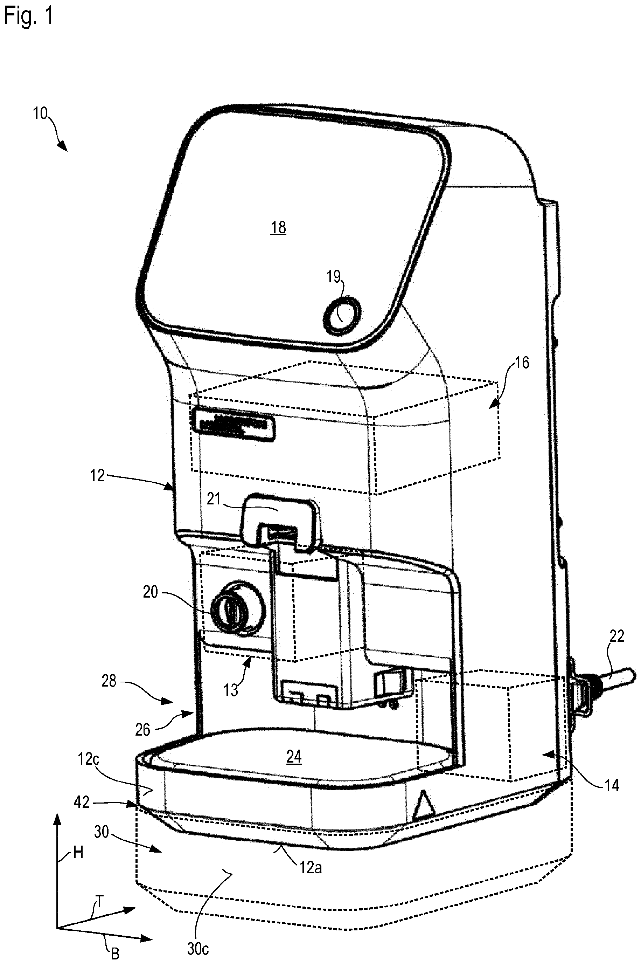

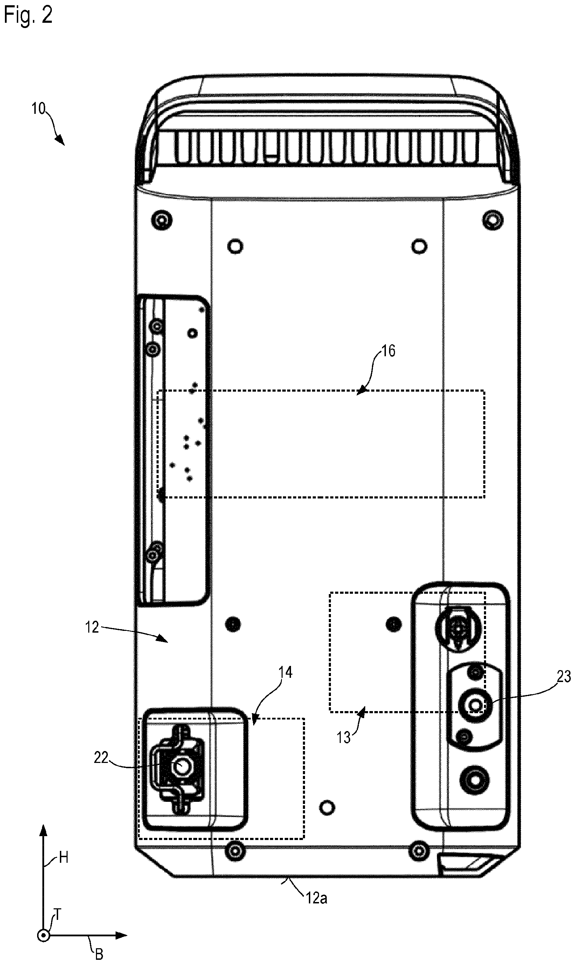

In den Figuren 1 und 2 ist eine Ausführungsform eines tragbaren Beatmungsgeräts gemäß der vorliegenden Erfindung allgemein mit 10 bezeichnet. Das Beatmungsgerät 10 umfasst ein Gehäuse 12, in welchem eine Atemgas-Fördervorrichtung 13 in Gestalt eines Gebläses, eine erste netzbasierte Stromversorgung 14, fachüblich auch als "Netzteil" bezeichnet, eine Steuervorrichtung 16 und eine Eingabe/Ausgabevorrichtung 18 aufgenommen ist. Vorrichtungen im Inneren des Gehäuses 12 sind lediglich strichliniert dargestellt.

Die Atemgas-Fördervorrichtung 13 saugt als Gebläse Umgebungsluft durch eine Ansaugöffnung (nicht dargestellt) an und gibt diese an einem Atemgas-Gehäuseausgang 20 an eine an den Atemgas-Gehäuseausgang 20 angeschlossene, jedoch vorliegend nicht dargestellte Atemgasleitung ab. Die Atemgas-Fördervorrichtung 13 erzeugt ausreichend Überdruck um die angesaugte Umgebungsluft nicht nur bis zum Atemgas-Gehäuseausgang 20, sondern durch die an diesen angeschlossene Atemgasleitung bis zum Patienten zu fördern.

Im vorliegend dargestellten Beispielfall öffnet der Atemgas-Gehäuseausgang 20 in einen in den Figuren nicht dargestellten Flüssigkeitsvorratsbehälter einer weiter unten erläuterten Befeuchtungsvorrichtung.

Ein Anschluss eines - bevorzugt zur Vermeidung von Kondensation beheizbaren - zum Patienten führenden Atemgasschlauchs kann an der Anschlussformation 21 erfolgen, von wo aus inspiratorisches Atemgas nach Durchgang durch den Flüssigkeitsvorratsbehälter der Befeuchtungsvorrichtung dem Patienten zugeführt wird.

Das Beatmungsgerät 10 des Ausführungsbeispiels ist ein sogenanntes "High-Flow" Beatmungsgerät 10, welches unabhängig vom Beatmungsrhythmus des beatmeten Patienten dem beatmeten Patienten kontinuierlich inspiratorisches Atemgas zuführt, auch während dessen Exspirationsphase. Die Erfindung ist jedoch nicht auf diesen Gerätetyp beschränkt.

Die Steuervorrichtung 16 steht mit der Atemgas-Fördervorrichtung 13, der ersten Stromversorgung 14 und der Eingabe/Ausgabevorrichtung 18 signalübertragend in Verbindung, sodass die Steuervorrichtung 16 an der Eingabe/Ausgabevorrichtung 18 eingegebene Steuerbefehle oder/und Parameter zur weiteren Verarbeitung erhält sowie Steuerbefehle an die Atemgas-Fördervorrichtung 13, an die erste Stromversorgung 14 und an die Eingabe/Ausgabevorrichtung 18 übertragen kann. Neben der Möglichkeit, Steuerbefehle zu übertragen, kann die Steuervorrichtung 16 über die signalübertragende Verbindung Betriebsparameter der Atemgas-Fördervorrichtung 13, der ersten Stromversorgung 14 und der Eingabe/Ausgabevorrichtung 18 erfassen und so deren Betrieb überwachen. Ebenso kann die Steuervorrichtung 16 an die Eingabe/Ausgabevorrichtung 18 Daten übertragen, um diese an der Eingabe/Ausgabevorrichtung 18 nach außen wahrnehmbar anzuzeigen bzw. auszugeben.

Die erste Stromversorgung 14 ist durch ein Netzkabel 22 mit einer Netzspannungsquelle (nicht dargestellt), etwa einer Steckdose, zur Verbindung mit einem öffentlichen Stromnetz verbindbar.

Mit 23 ist eine Anschlussformation zum Anschluss einer weiteren Atemgasquelle mit einem vorzugsweise von der Umgebungsluft verschiedenen weiteren Atemgas bezeichnet. So kann die von der Atemgas-Fördervorrichtung 13 angesaugte Umgebungsluft mit einem weiteren Gas im Beatmungsgerät 10 vor der Ausgabe an den Atemgas-Gehäuseausgang 20 vermischt werden.

Die Eingabe/Ausgabevorrichtung 18 ist bevorzugt um eine zur Breitenrichtung B parallele Anstellachse geneigt, sodass ihre Eingabeoberfläche 18a für Bedienpersonen leicht erreichbar ist. Die Eingabeoberfläche 18a kann beispielsweise ein berührungssensitiver Bildschirm, also ein sogenannter "Touch-Screen" sein. Alternativ oder zusätzlich zu lediglich abgegrenzten Schaltflächen mit Funktionszuordnung, wie sie für berührungssensitive Bildschirme typisch sind, kann die Eingabe/Ausgabevorrichtung 18 auch einen oder mehrere Schalter, wie Tastschalter, Drehschalter und dergleichen umfassen. Beispielhaft sei der Ein/Aus-Schalter 19 erwähnt, der als mechanischer Tastschalter ausgebildet ist.

Das Beatmungsgerät 10 ist in den Figuren 1 und 2 für einen Beatmungsbetrieb bereit orientiert dargestellt.

Als weitere Funktionseinrichtung weist das Beatmungsgerät 10 eine Heizplatte 24 einer Befeuchtungsvorrichtung 26 auf, deren nicht dargestellter Flüssigkeitsvorrat in die Ausnehmung 28 an der Vorderseite des Gehäuses 12 einsetzbar ist. Auch die Heizplatte 24 der Befeuchtungsvorrichtung 26 wird durch die Steuervorrichtung 16 gesteuert. Die Steuervorrichtung 16 kann hierzu mit einem oder mehreren Feuchtigkeitssensoren signalübertragend verbunden sein, welche eine Feuchtigkeit des von der Atemgas-Fördervorrichtung 13 geförderten inspiratorischen Beatmungsgases nach Durchgang durch die Befeuchtungsvorrichtung 26 und gegebenenfalls auch vor dem Durchgang durch diese erfassen und an die Steuervorrichtung 16 übertragen.

Da die Befeuchtungsvorrichtung 26 mit dem Flüssigkeitsvorrat ein vergleichsweise hohes Gewicht pro Volumeneinheit aufweist, ist die Befeuchtungsvorrichtung 26 in Höhenrichtung H möglichst weit unten im Gehäuse 12 angeordnet, um einen möglichst tiefen Schwerpunkt des Beatmungsgeräts 10 zu erzielen. Aus diesem Grunde ist auch die ebenfalls ein hohes Gewicht pro Volumeneinheit aufweisende erste Stromversorgung 14 möglichst weit unten im Gehäuse 12 angeordnet. Vorzugsweise liegen der Flüssigkeitsvorrat der Befeuchtungsvorrichtung 26 und die erste Stromversorgung im unteren Drittel des Gehäuses 12.

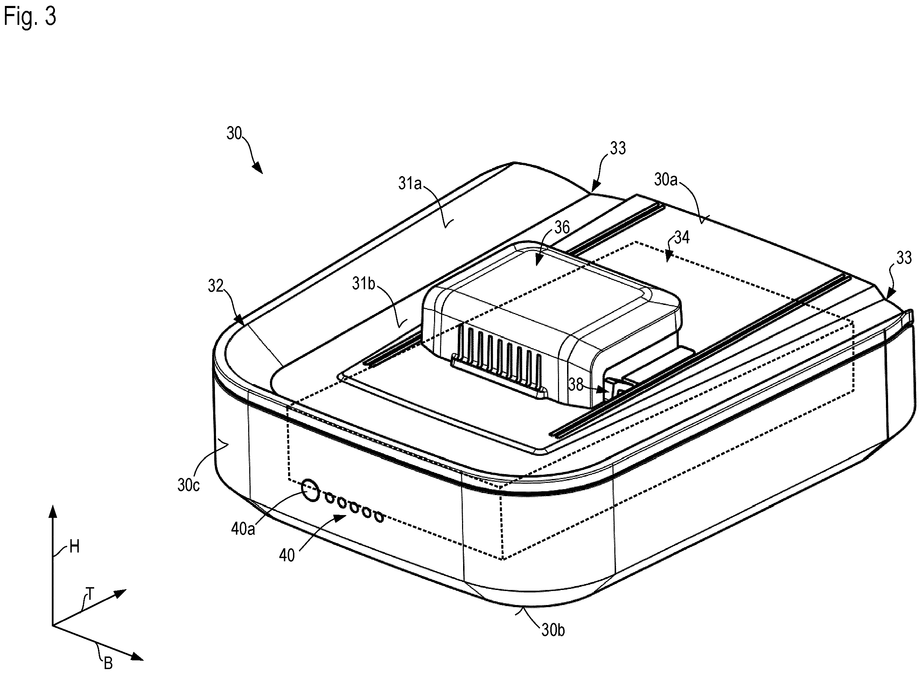

Das Beatmungsgerät 10 weist außerdem eine in Figur 3 gezeigte zweite Stromversorgung 30 auf, welche in der dargestellten bevorzugten Ausführungsform ein vom Gehäuse 12 des Beatmungsgeräts 10 gesondert ausgebildetes Stromversorgungsgehäuse 32 aufweist. Die zweite Stromversorgung 30 ist keine netzbasierte Stromversorgung wie die erste Stromversorgung 14, sondern weist einen elektrischen Energiespeicher 34 auf, welcher nachfolgend auch als "Stromspeicher" bezeichnet ist.

Der Stromspeicher 34 ist bevorzugt ein mehrfach wiederaufladbarer Akkumulator, wie beispielsweise ein Lithium-Ionen-Akkumulator. Der Stromspeicher kann jedoch auch eine andere Art von Akkumulatoren oder/und wenigstens einen Kondensator als elektrische Energiespeicher aufweisen.

Da das Beatmungsgerät 10 mit und ohne die zweite Stromversorgung 30 betrieben werden kann, ist die zweite Stromversorgung 30 in Figur 1 lediglich strichliniert angedeutet.

Die zweite Stromversorgung 30 kann an die Unterseite 12a des Gehäuses 12 des Beatmungsgeräts 10 körperlich derart lösbar angekoppelt werden, dass die zweite Stromversorgung 30 im angekoppelten Zustand gemeinsam mit dem Gehäuse 12 bewegbar ist. Im abgekoppelten Zustand kann das Gehäuse 12 auf seiner Unterseite 12a aufliegen.

Die zweite Stromversorgung 30 weist an ihrer Oberseite 30a eine Kopplungsformation 36 auf, welche Strom übertragend mit einer Kopplungsgegenformation an der Unterseite 12a des Gehäuses 12 mechanisch formschlüssig in Eingriff bringbar ist. Wie Figur 3 zeigt, ist die Kopplungsformation 36 als Vorsprung ausgebildet. Die nicht dargestellte Kopplungsgegenformation an der Unterseite 12a des Gehäuses 12 ist daher in Gestalt einer Ausnehmung ausgebildet. Eine Verriegelungsvorrichtung 38 verriegelt die zweite Stromversorgung 30 in einem für eine Übertragung von elektrischer Energie von der zweiten Stromversorgung 30 an das übrige Beatmungsgerät 10 bereiten Zustand. Die Verriegelungsvorrichtung 38 kann selbsttätig verrastend vorgespannt sein und kann beispielsweise durch einen Betätigungsangriff auf der Unterseite 30b der zweiten Stromversorgung 30 für ein Abkoppeln der zweiten Stromversorgung 30 vom Gehäuse 12 des Beatmungsgeräts 10 entriegelbar sein.

An seiner Vorderseite kann im Stromversorgungsgehäuse 32 eine Ladezustandsanzeige 40 angeordnet sein, welche beispielsweise eine Mehrzahl von LEDs umfassen kann, von welchen eine umso größere Anzahl beleuchtet ist, je stärker der Stromspeicher 34 geladen ist. Zusätzlich oder alternativ kann die Ladezustandsanzeige 40 ihre Leuchtfarbe ändern, etwa von grün auf rot und umgekehrt, um zusätzliche Information zu vermitteln, wie beispielsweise das unmittelbare bevorstehen eines Betriebsendes der zweiten Stromversorgung 30 aufgrund von Entleerung des Stromspeichers 34. Mittels eines Tastschalters 40a kann die Ladezustandsanzeige 40 aktiviert werden, die zu Zwecken der Energieeinsparung nur auf Betätigung des Tastschalters 40a und nur für eine vorbestimmte kurze Zeit von wenigen Sekunden den Ladezustand des Stromspeichers 34 anzeigt.

Dann, wenn die zweite Stromversorgung 30 am Gehäuse 12 betriebsbereit angeordnet ist, kann die Steuervorrichtung 16 auch den Betrieb der zweiten Stromversorgung 30 steuern. Beispielsweise kann durch Bedieneingabe festgelegt werden, ob dann, wenn beide Stromversorgungen 14 und 30 als Stromlieferant zur Verfügung stehen, das Beatmungsgerät durch die erste Stromversorgung 14 oder durch die zweite Stromversorgung 30 mit elektrischer Energie versorgt werden soll. Steht die erste Stromversorgung 14 nicht zur Verfügung, etwa weil ein Stromausfall vorliegt oder weil die erste Stromversorgung 14 defekt ist oder weil das Netzkabel 22 fehlt, wird das Beatmungsgerät 10 automatisch durch die zweite Stromversorgung 30 mit elektrischer Energie versorgt.

Dann, wenn die erste Stromversorgung 14 als Stromlieferant zur Verfügung steht, kann die Steuervorrichtung 16 ein Aufladen des Stromspeichers 34 durch die erste Stromversorgung 14 veranlassen, sofern nach Versorgung der betriebswichtigen Funktionsvorrichtungen, wie der Atemgas-Fördervorrichtung 13, und der Steuervorrichtung 16 selbst, gegebenenfalls auch der Eingabe/Ausgabevorrichtung 18 oder/und der Befeuchtungsvorrichtung 26 ausreichend Reserven für ein Aufladen des Stromspeichers 34 zur Verfügung stehen.

Die zweite Stromversorgung 30 wirkt am Beatmungsgerät 10 wie eine unterbrechungsfreie Stromversorgung, d. h. ein Ausfall der Stromversorgung des Beatmungsgeräts 10 durch die erste Stromversorgung 14 wird in so kurzer Zeit durch die zweite Stromversorgung 30 kompensiert, dass es zu keiner nennenswerten Unterbrechung des Beatmungsbetriebs des Beatmungsgeräts 10 kommt. In der Praxis bedeutet dies, dass die zweite Stromversorgung 30 in spätestens 50 ms nach einem Ausfall der ersten Stromversorgung 14 elektrische Energie an die bisher von der ersten Stromversorgung 14 versorgten Funktionsvorrichtungen abgibt.

Die zweite Stromversorgung 30 ist deshalb an die Unterseite 12a des Gehäuses 12 anbringbar, da die zweite Stromversorgung 30 bezogen auf ihr Volumen ein überdurchschnittlich hohes Gewicht aufweist und somit zu einem gewünscht tiefen Schwerpunkt des gesamten Beatmungsgeräts 10 einschließlich der zweiten Stromversorgung 30 beitragen kann.

Wie Figur 1 andeutet, schließt sich eine um eine zur Höhenrichtung H parallele Höhenachse umlaufende Mantelfläche 30c im Wesentlichen bündig an eine ebensolche Mantelfläche 12c des Gehäuses 12 an seinem unteren Endbereich an, etwa zwischen der Heizplatte 24 und der Unterseite 12a des Gehäuses 12.

Das Gehäuse 12 ist ein Turmgehäuse, d. h. es hat in Höhenrichtung H eine wesentlich größere Abmessung als in Breitenrichtung B und in Tiefenrichtung T. Im dargestellten Beispiel ist die Abmessung in Breitenrichtung in dem die Mantelfläche 12c aufweisenden Bereich des Gehäuses 12 etwa gleich groß wie die Abmessung in Tiefenrichtung. Zwischen der Mantelfläche 12c und einer Aufstandsfläche des Gehäuses 12 kann eine Schräge ausgebildet sein, welche als komplementäre Ausbildung einer Schräge 31a vorzugsweise auch an der Oberseite 30a der zweiten Stromversorgung 30 bzw. des Stromversorgungsgehäuse 32 ausgebildet sein kann. Somit kann die zweite Stromversorgung 30 mit sehr geringem Bewegungsspiel hochgradig stabil und mit großer Festigkeit lösbar mit der Unterseite 12a des Gehäuses 12 gekoppelt werden.

Die Schräge 31a dient außerdem als der oben genannte erste Flächenabschnitt der Oberseite 30a zur Ableitung von Flüssigkeit von der Außenwand des Beatmungsgeräts zu einem vorbestimmten Ableitort 33. Im vorliegenden Fall existieren zwei Ableitorte 33.

Die Schräge 31a ist in Richtung zur Körpermitte der zweiten Stromversorgung 30 hin geneigt. Die Schräge 31a ist außerdem mehrfach um eine zur Höhenrichtung parallele Krümmungsachse gekrümmt, so dass sich die Schräge 31a sowohl über die der in Figur 2 gezeigte Rückseite entgegengesetzte Vorderseite als auch über die zwei an die Vorderseite anschließenden Seiten des Beatmungsgeräts 10 erstrecken kann.

An den geodätisch tieferliegenden Rand der Schräge 31a schließt sich ein zweiter Flächenbereich 31b an, der derart geneigt ist, dass die Ableitorte 33 die geodätisch tiefsten Orte des Flächenbereichs 31b sind, sodass sich von der Schräge 31a in den zweiten Flächenbereich 31b überströmende Flüssigkeit weiter schwerkraftgetrieben zu den Ableitorten 33 strömt.

Die Schräge 31a beginnt unmittelbar am äußeren Rand der Oberseite 30a des Stromversorgungsgehäuses 32, sodass Flüssigkeit, die an der Mantelfläche 12c des Gehäuses 12 schwerkraftgetrieben zur Trennfuge 42 zwischen Gehäuse 12 und Stromversorgungsgehäuse 32 herabrinnt, bei Erreichen der Trennfuge zunächst durch die Schräge 31a und anschließend durch den zweiten Flächenbereich 31b von der Trennfuge 42 zu den Ableitorten 33 geleitet wird.

In dem dargestellten Ausführungsbeispiel verläuft die Höhenrichtung H parallel zur Schwerkraftwirkungsrichtung, jedoch in entgegengesetzter Richtung. Wie insbesondere in Figur 1 erkennen ist, liegt die mit dem Gehäuse 12 gekoppelte zweite Stromversorgung 30 vollständig in einem längs der Schwerkraftwirkungsrichtung projizierten Grundrisses des Gehäuses 12 und ragt seitlich nicht über diesen hervor. Die zweite Stromversorgung 30 bleibt auch nicht hinter diesem projizierten Grundriss des Gehäuses 12 zurück, sondern entspricht in ihrer Ausdehnung in einer durch die Breitenrichtung B und die Tiefenrichtung T aufgespannten Ebene im Wesentlichen der Grundrissfläche des Gehäuses 12.

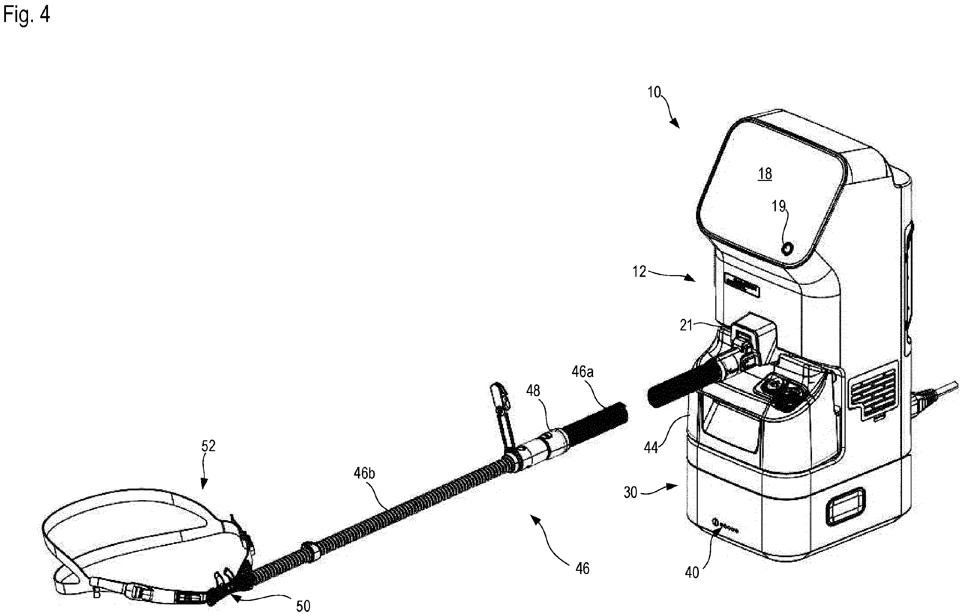

In Figur 4 ist das Beatmungsgerät 10 von Figur 1 mit einem Flüssigkeitsvorratsbehälter 44 dargestellt, welcher in die Ausnehmung 28 eingesetzt ist. Mit der Anschlussformation 21 ist ein Beatmungsschlauch 46 gekoppelt, welcher durch die Befeuchtungsvorrichtung 26 konditioniertes Atemgas zu einem nicht dargestellten Patienten leitet.

An dem vom Beatmungsgerät 10 fernliegenden Ende eines ersten Abschnitts 46a des Beatmungsschlauchs 46 ist ein Temperatursensor 48 angeordnet, welcher die Temperatur des Atemgases im Beatmungsschlauch 46, insbesondere am Ende des ersten Abschnitts 46a, erfasst und an die Steuervorrichtung 16 überträgt, entweder per Funk oder durch ein im oder am Beatmungsschlauch 46 geführtes Kabel, welches beim Koppeln des Beatmungsschlauchs 46 mit der Anschlussformation 21 bevorzugt automatisch signalübertragend mit der Steuervorrichtung 16 verbunden wird.

Ausgehend von dem Temperatursignal des Temperatursensors 48 kann die Steuervorrichtung 16 insbesondere dann, wenn das Beatmungsgerät 10 nur durch die zweite Stromversorgung 30 mit elektrischer Energie versorgt wird, den Betrieb der Heizplatte 24, aber auch einer etwaig im Beatmungsschlauch 46 vorgesehenen Heizvorrichtung, derart steuern, dass das Atemgas am Temperatursensor 48 eine Temperatur innerhalb eines vorbestimmten Temperaturbereichs aufweist, etwa innerhalb eines Temperaturbereichs von 30 °C bis 40 °C, bevorzugt von 33 °C bis 38 °C, besonders bevorzugt von 35 °C.

Der erste, dem Beatmungsgerät 10 näher gelegene Abschnitt 46a des Beatmungsschlauch 46 ist in Figur 4 verkürzt dargestellt.

An den ersten Abschnitt 46a schließt sich ein zweiter Abschnitt 46b des Beatmungsschlauchs 46 an, an dessen Ende eine Nasenbrille 50 angeordnet ist, die über ein Kopfband 52 am Kopf des Patienten in an sich bekannter Weise festgelegt werden kann. Über die Nasenbrille 50 kann das Atemgas kontinuierlich an den Patienten abgegeben werden.

Die Erfindung wird durch die folgenden beigefügten Ansprüche definiert.

## Patentansprüche

1. Tragbares Beatmungsgerät (10) zur Atemgaszufuhr zu einem Lebewesen, umfassend:
- ein Gehäuse (12),
- eine Atemgas-Fördervorrichtung (13), welche dazu ausgebildet ist, inspiratorisches Atemgas zu einem Atemgas-Gehäuseausgang (20) des Gehäuses (12) zu fördern,
- eine Eingabe/Ausgabevorrichtung (18) zur Eingabe von Steuerbefehlen und zur Ausgabe von Information,
- eine Steuervorrichtung (16), welche signalübertragend mit der Eingabe/- Ausgabevorrichtung (18) und mit der Atemgas-Fördervorrichtung (13) verbunden ist,
- eine erste, netzbasierte Stromversorgung (14), welche zur Strom übertragenden Verbindung mit einer bezüglich des Beatmungsgeräts (10) externen Netzspannungsquelle ausgebildet ist und welche zur Versorgung der Steuervorrichtung (16), der Eingabe/Ausgabevorrichtung (18) und der Atemgas-Fördervorrichtung (13) mit Strom ausgebildet und angeordnet ist,
- eine zweite, speicherbasierte Stromversorgung (30), welche einen Stromspeicher (34) zur Speicherung elektrischer Energie aufweist, und welche wenigstens zur Versorgung der Atemgas-Fördervorrichtung (13) mit Strom ausgebildet ist,
- eine Befeuchtungsvorrichtung (26), welche dazu ausgebildet ist, das inspiratorische Atemgas zu befeuchten, wobei die Befeuchtungsvorrichtung (26) sowohl durch die erste (14) als auch durch die zweite Stromversorgung (16) mit Strom versorgbar ist,
wobei die Atemgas-Fördervorrichtung (13), die Eingabe/Ausgabevorrichtung (18) und die Steuervorrichtung (16) in dem Gehäuse (12) aufgenommen sind, wobei die zweite Stromversorgung (30) ein vom Gehäuse (12) des Beatmungsgeräts (10) gesondertes Stromversorgungsgehäuse (32) aufweist, welches körperlich lösbar mit dem Gehäuse (12) des Beatmungsgeräts (10) zur gemeinsamen Bewegung koppelbar ist,
wobei die zweite Stromversorgung (30) bei Betrachtung des betriebsbereit hergerichteten Beatmungsgeräts (10) unter der Befeuchtungsvorrichtung (26) angeordnet ist,
**dadurch gekennzeichnet, dass** die erste Stromversorgung (14) in dem Gehäuse (12) des Beatmungsgeräts (10) aufgenommen ist, wobei das Beatmungsgerät (10) ausschließlich durch die erste Stromversorgung (14) mit elektrischer Energie versorgbar ist, wenn die zweite Stromversorgung (30) vom Gehäuse (12) des Beatmungsgeräts (10) getrennt ist.

2. Beatmungsgerät (10) nach Anspruch 1,
**dadurch gekennzeichnet, dass** die zweite Stromversorgung (30) auch zur Versorgung der Steuervorrichtung (16) und der Eingabe/Ausgabevorrichtung (18) mit Strom ausgebildet ist.

3. Beatmungsgerät (10) nach einem der vorhergehenden Ansprüche,
**dadurch gekennzeichnet, dass** der Stromspeicher (34) der zweiten Stromversorgung (30) durch die erste Stromversorgung (14) aufladbar ist, wobei bevorzugt die Steuervorrichtung (16) dazu ausgebildet ist, einen Ladevorgang des Stromspeichers (34) durch die erste Stromversorgung (14) zu steuern.

4. Beatmungsgerät (10) nach einem der vorhergehenden Ansprüche,
**dadurch gekennzeichnet, dass** die zweite Stromversorgung (30) als unterbrechungsfreie Stromversorgung für den Fall eines Endes einer Stromversorgung des Beatmungsgeräts durch die erste Stromversorgung (14) ausgebildet ist.

5. Beatmungsgerät (10) nach einem der vorhergehenden Ansprüche,
**dadurch gekennzeichnet, dass** die zweite Stromversorgung (30) bei betriebsbereiter Aufstellung des Beatmungsgeräts (10) in den untersten 20 % der Gesamthöhenerstreckung des Beatmungsgeräts (10) mit zweiter Stromversorgung (30) angeordnet ist, vorzugsweise die unterste Funktionsvorrichtung am Beatmungsgerät (10) mit zweiter Stromversorgung (30) ist.

6. Beatmungsgerät (10) nach einem der vorhergehenden Ansprüche,
**dadurch gekennzeichnet, dass** am Stromversorgungsgehäuse (32) Ableitflächen (31a, 31b) ausgebildet sind, die darauf auftreffende Flüssigkeit gezielt zu wenigstens einem definierten Ableitort (33) ableiten.

7. Beatmungsgerät (10) nach einem der vorhergehenden Ansprüche,
**dadurch gekennzeichnet, dass** das Stromversorgungsgehäuse (32) - bei Betrachtung einer betriebsbereiten Aufstellung des Beatmungsgeräts (10) - vollständig innerhalb eines in Schwerkraftwirkungsrichtung projizierten Grundrisses des Gehäuses (12) des Beatmungsgeräts (10) aufgenommen ist.

8. Beatmungsgerät (10) nach einem der vorhergehenden Ansprüche,
**dadurch gekennzeichnet, dass** das Gehäuse (12) ein Turmgehäuse ist, dessen größte Abmessung bei betriebsbereiter Aufstellung die Höhenabmessung ist.

9. Beatmungsgerät (10) nach einem der vorhergehenden Ansprüche,
**dadurch gekennzeichnet, dass** das Beatmungsgerät (10) eine beheizbare Atemgasleitung (46) umfasst, die vom Beatmungsgerät (10) zum Patienten führt.

10. Beatmungsgerät (10) nach Anspruch 9,
**dadurch gekennzeichnet, dass** die Steuervorrichtung (16) dazu ausgebildet ist, dann, wenn der Ladezustand des Stromspeichers (34) einen ersten Grenz-Ladezustand unterschreitet, eine Verdampfungsvorrichtung (24) der Befeuchtungsvorrichtung (26) stillzusetzen, und dann, wenn der Ladezustand einen zweiten Grenz-Ladezustand unterschreitet, welcher betragsmäßig niedriger ist als der erste Grenz-Ladezustand, eine Heizvorrichtung der Atemgasleitung (46) stillzusetzen.

## Claims

1. Portable respiratory device (10) for supplying respiratory gas to a living organism, comprising:
- A housing (12),
- A respiratory gas conveying device (13) which is configured to convey inspiratory respiratory gas to a respiratory gas housing outlet (20) of the housing (12),
- An input/output device (18) for the input of control commands and for the output of information,
- A control device (16) which is connected for signal transmission with the input/output device (18) and with the respiratory gas conveying device (13),
- A first, power grid-based power supply (14) which is configured for current-transmitting connection with a power grid voltage source external to the respiratory device (10) and which is configured and arranged for supplying the control device (16), the input/output device (18), and the respiratory gas conveying device (13) with current,
- A second, storage-based power supply (30) which exhibits an electricity storage device (34) for storing electrical energy and which is configured at least for supplying the respiratory gas conveying device (13) with current,
- A humidification device (26) which is configured to humidify the inspiratory respiratory gas, where the humidification device (26) can be supplied with current both by the first (14) and by the second power supply (30),
Where the respiratory gas conveying device (13), the input/output device (18), and the control device (16) are accommodated in the housing (12),
Where the second power supply (30) exhibits a power supply housing (32) separate from the housing (12) of the respiratory device (10) which is physically couplable in a detachable manner with the housing (12) of the respiratory device (10) for common movement,
Where when looking at the operationally ready respiratory device (10), the second power supply (30) is arranged under the humidification device (26),
**Characterized in that** the first power supply (14) is accommodated in the housing (12) of the respiratory device (10), where the respiratory device (10) can be supplied with electrical energy solely by the first power supply (14) when the second power supply (30) is separated from the housing (12) of the respiratory device (10).

2. Respiratory device (10) according to Claim 1,
**Characterized in that** the second power supply (30) is also configured for supplying the control device (16) and the input/output device (18) with current.

3. Respiratory device (10) according to one of the preceding Claims,
**Characterized in that** the electricity storage device (34) of the second power supply (30) is chargeable by the first power supply (14), where preferably the control device (16) is configured to control a charging process of the electricity storage device (34) by the first power supply (14).

4. Respiratory device (10) according to one of the preceding Claims,
**Characterized in that** the second power supply (30) is configured as an interruption-free power supply for the case of an end of a power supply to the respiratory device by the first power supply (14).

5. Respiratory device (10) according to one of the preceding Claims,
**Characterized in that** during operational positioning of the respiratory device (10), the second power supply (30) is arranged in the lowest 20% of the overall height extension of the respiratory device (10) with second power supply (30), preferably being the lowest functional device at the respiratory device (10) with second power supply (30).

6. Respiratory device (10) according to one of the preceding Claims,
**Characterized in that** at the power supply housing (32) there are configured drainage surfaces (31a, 31b) which in a targeted manner drain liquid impinging on them to at least one defined drainage location (33).

7. Respiratory device (10) according to one of the preceding Claims,
**Characterized in that** the power supply housing (32) - when looking at an operational positioning of the respiratory device (10) - is completely accommodated inside a footprint of the housing (12) of the respiratory device (10) projected in the gravitational direction.

8. Respiratory device (10) according to one of the preceding Claims,
**Characterized in that** the housing (12) is a tower housing whose largest dimension during operational positioning is the vertical dimension.

9. Respiratory device (10) according to one of the preceding Claims,
**Characterized in that** the respiratory device (10) comprises a heatable respiratory gas line (46) which leads from the respiratory device (10) to the patient.

10. Respiratory device (10) according to Claim 9,
**Characterized in that** the control device (16) is configured, when the charging state of the electricity storage device (34) drops below a first charging state limit, to shut down an evaporation device (24) of the humidification device (26), and when the charging state drops below a second charging state limit which is quantitatively lower than the first charging state limit, to shut down a heating device of the respiratory gas line (46).

## Revendications

1. Dispositif portatif de respiration (10) pour fournir du gaz respiratoire à un être vivant, comprenant :
- un boîtier (12),
- un dispositif de transport de gaz respiratoire (13) adapté pour transporter du gaz respiratoire inspiratoire vers une sortie de boîtier de gaz respiratoire (20) du boîtier (12),
- un dispositif d'entrée/sortie (18) pour entrer des instructions de commande et pour sortir des informations,
- un dispositif de commande (16) qui est relié par transmission de signaux au dispositif d'entrée/sortie (18) et au dispositif de transport de gaz respiratoire (13),
- une première alimentation électrique (14) basée sur le réseau, qui est conçue pour la liaison de transmission de courant avec une source de tension de réseau externe par rapport au respirateur (10) et qui est conçue et disposée pour alimenter en courant le dispositif de commande (16), le dispositif d'entrée/sortie (18) et le dispositif de transport de gaz respiratoire (13),
- une deuxième alimentation électrique (30) basée sur un accumulateur d'énergie, qui présente un accumulateur d'énergie (34) pour le stockage d'énergie électrique, et qui est conçue au moins pour l'alimentation en courant du dispositif de transport de gaz respiratoire (13),
- un dispositif d'humidification (26) qui est conçu pour humidifier le gaz respiratoire inspiratoire, le dispositif d'humidification (26) pouvant être alimenté en courant tant par la première (14) que par la deuxième alimentation électrique (16),
dans lequel le dispositif de transport de gaz respiratoire (13), le dispositif d'entrée/sortie (18) et le dispositif de commande (16) sont logés dans le boîtier (12), dans lequel la deuxième alimentation électrique (30) comprend un boîtier d'alimentation électrique (32) séparé du boîtier (12) du respirateur (10) qui peut être couplé de manière physiquement détachable au boîtier (12) du respirateur (10) pour un mouvement commun,
dans lequel la seconde alimentation électrique (30) est située sous le dispositif d'humidification (26) lorsque l'on observe le respirateur (10) prêt à l'emploi,
**caractérisé en ce que** la première alimentation électrique (14) est logée dans le boîtier (12) du respirateur (10), le respirateur (10) pouvant être alimenté en énergie électrique exclusivement par la première alimentation électrique (14) lorsque la deuxième alimentation électrique (30) est séparée du boîtier (12) du respirateur (10).

2. Respirateur (10) selon la revendication 1,
**caractérisé en ce que** la deuxième alimentation électrique (30) est également configurée pour alimenter en électricité le dispositif de commande (16) et le dispositif d'entrée/sortie (18).

3. Respirateur (10) selon l'une des revendications précédentes,
**caractérisé en ce que** l'accumulateur d'énergie (34) de la deuxième alimentation électrique (30) peut être chargé par la première alimentation électrique (14), le dispositif de commande (16) étant de préférence conçu pour commander une opération de charge de l'accumulateur d'énergie (34) par la première alimentation électrique (14).

4. Respirateur (10) selon l'une des revendications précédentes,
**caractérisé en ce que** la deuxième alimentation électrique (30) est conçue comme une alimentation électrique sans coupure en cas de fin d'une alimentation électrique du respirateur par la première alimentation électrique (14).

5. Respirateur (10) selon l'une des revendications précédentes,
**caractérisé en ce que** la deuxième alimentation électrique (30) est disposée dans les 20% les plus bas de l'extension en hauteur totale du respirateur (10) avec une deuxième alimentation électrique (30) lorsque le respirateur (10) est en position opérationnelle, de préférence est le dispositif fonctionnel le plus bas sur le respirateur (10) avec une deuxième alimentation électrique (30).

6. Respirateur (10) selon l'une des revendications précédentes,
**caractérisé en ce que** des surfaces de dérivation (31a, 31b) sont formées sur le boîtier d'alimentation électrique (32), lesquelles dérivent de manière ciblée le liquide qui les touche vers au moins un lieu de dérivation défini (33).

7. Respirateur (10) selon l'une des revendications précédentes,
**caractérisé en ce que** le boîtier d'alimentation électrique (32) est - lorsque le respirateur (10) est observé en position opérationnelle - entièrement logé à l'intérieur d'un plan du boîtier (12) du respirateur (10) projeté dans la direction d'action de la pesanteur,.

8. Respirateur (10) selon l'une des revendications précédentes,
**caractérisé en ce que** le boîtier (12) est un boîtier en forme de tour dont la plus grande dimension, lorsqu'il est en position opérationnelle, est la dimension en hauteur.

9. Respirateur (10) selon l'une des revendications précédentes,
**caractérisé en ce que** le respirateur (10) comprend une conduite de gaz respiratoire (46) pouvant être chauffée, qui mène du respirateur (10) au patient.

10. Respirateur (10) selon la revendication 9,
**caractérisé en ce que** le dispositif de commande (16) est conçu pour arrêter un dispositif d'évaporation (24) du dispositif d'humidification (26) lorsque l'état de charge de l'accumulateur d'énergie (34) passe en dessous d'un premier état de charge limite, et pour arrêter un dispositif de chauffage de la conduite de gaz respiratoire (46) lorsque l'état de charge passe en dessous d'un deuxième état de charge limite, qui est inférieur en valeur au premier état de charge limite.
